Europäisches Patentamt

European Patent Office

Office européen des brevets

(11) Publication number: **0 308 704**
**A1**

## (12) EUROPEAN PATENT APPLICATION

(21) Application number: 88114356.4

(22) Date of filing: 02.09.88

(51) Int. Cl.⁴: **G01N 33/545 , G01N 33/541 , G01N 33/535 , G01N 33/52**

The title of the invention has been amended (Guidelines for Examination in the EPO, A-III, 7.3).

(30) Priority: 03.09.87 JP 220796/87
03.09.87 JP 220797/87

(43) Date of publication of application:
29.03.89 Bulletin 89/13

(84) Designated Contracting States:
DE GB

(71) Applicant: FUJI PHOTO FILM CO., LTD.
210 Nakanuma Minami Ashigara-shi
Kanagawa 250-01(JP)

(72) Inventor: Kitajima, Masao c/o Fuji Photo
Film Co., Ltd 11-46, Senzui, 3-chome
Asaka-shi Saitama(JP)
Inventor: Kageyama, Shigeki c/o Fuji Photo
Film Co., Ltd. 11-46, Senzui, 3-chome
Asaka-shi Saitama(JP)
Inventor: Hiraoka, Toshikage c/o Fuji Photo
Film Co., Ltd. 11-46, Senzui, 3-chome
Asaka-shi Saitama(JP)

(74) Representative: Kraus, Walter, Dr. et al
Patentanwälte Kraus, Weisert & Partner
Thomas-Wimmer-Ring 15
D-8000 München 22(DE)

(54) Heterogeneous enzymimmunoassay.

(57) The immunoassay of the invention is characterized by that, in a heterogeneous EIA, a definite amount of the liquid phase containing the free portion is taken out after the antigen-antibody reaction, and the enzyme activity of the liquid phase is measured using a dry-type chemical analytical film containing all reagents necessary for measuring the enzyme activity.

In the immunoassay of the invention, an antibody or antigen is physically and/or chemically immobilized to a suitable porous membrane such as cellulose acetate porous membrane prepared by phase-inversion process, the antigen or antibody contained in a sample is allowed to react with the above immobilized antibody or antigen, respectively, in the presence of an enzyme-labeled antigen or antibody, a definite amount of the liquid phase is taken out, and its enzyme activity is measured using a dry-type chemical analytical film. In the above immunoassay, a definite amount of an enzyme-labeled antigen or an enzyme-labeled antibody may be incorporated into the porous membrane containing the immobilized antibody or antigen prior to the immunological reaction involving the antigen or antibody contained in a sample.

The immunoassay does not require troublesome and time-consuming operations such as the transfer of a reaction product to another vessel, the preparation of reagents, the addition of substrate and terminator and the measurement of the absorbance of the reaction solution, in heterogeneous EIA.

EP 0 308 704 A1

## IMMUNOASSAY

## BACKGROUND OF THE INVENTION

### Field of the Invention

The analyses of medicines and metabolites contained in a body fluid are very useful for the diagnosis of disease, the judgement on remedy course of disease and the like, and they are important in clinical assay field. This invention relates to the immunoassay for measuring these substances.

### Description of the Prior Art

Many methods have been developed for detecting various substances derived from an organism, and a large group methodologically classified is immunoassay. The immunoassay is divided into radioimmunoassay, enzyme immunoassay, fluoroimmunoassay, etc. according to the kind of label substance. Enzyme immunoassay (EIA) determines an antigen or antibody by utilizing antigen-antibody reaction where enzyme activity is employed as a marker, i.e. label. EIA is classified into homogeneous EIA where the separation of the bound portion of antigen and antibody (bound portion) from unreacted portion (free portion) is not necessary and heterogeneous EIA where bound/free separation (B/F separation) is necessary. The EIA is explained in detail in Eiji Ishikawa "Koso Men-eki Sokutei-Ho (Enzyme Immunoassay) 2nd. Ed.", Igaku-Shoin, Japan, 1982. Heterogeneous EIA is not efficient because it requires troublesome operations, such as the transfer of a reaction product to another vessel, the preparation of reagents, the addition of substrate and terminator and the measurement of the absorbance of the reaction solution. In the past, B/F separation was carried out by using a separating agent, such as second antibody and centrifuging. These operations were troublesome, and required time. Thereafter, solid phase method has been developed, and centrifuging is no more necessary owing to the use of immobilized antigen or antibody where the antigen or antibody participating in the antigen-antibody reaction is bound to a solid phase carrier. However, the preparation of reagent solutions, addition of substrate and terminator, washing operation and the like are still necessary. In the solid phase method, the enzyme activity of the portion bound to the solid phase was, in general, measured after the antigen-antibody reaction. Measuring the enzyme activity of the free portion, i.e. liquid phase, is troublesome compared with measuring the enzyme activity of the solid phase, and a sufficient sensivity was not obtained, yet.

## SUMMARY OF THE INVENTION

An object of the invention is to provide an immunoassay capable of determining various antigenic substance such as medicines and metabolites in a body fluid, not requiring troublesome and time-consuming operations such as the transfer of a reaction product to another vessel, the preparation of reagents, the addition of substrate and terminator and the measurement of the absorlance of the reaction solution, in a heterogeneous EIA.

The present invention provides an immunoassay which has been achieved such an object. The immunoassay is characterized by that, in a heterogeneous EIA, a definite amount of the liquid phase containing the free portion is withdrawn after the antigen-antibody reaction, and the enzyme activity of the liquid phase is measured using a dry-type chemical analytical film containing all reagents necessary for measuring the enzyme activity.

In the immunoassay of the invention, an antibody or antigen is physically and/or chemically immobilized to a suitable porous membrane such as cellulose acetate porous membrane prepared by phase-inversion process.

According to the present invention, the antigen contained in the sample is allowed to react with the immobilized antibody in the presence of a definite amount of the enzyme-labeled antigen or antibody, or is allowed to react at first with the immobilized antibody, thereafter with a definite amount of the enzyme-labeled antibody.

Alternatively, according to the present invention, the antibody contained in the sample is allowed to react with the immobilized antigen in the presence of a definite amount of the enzyme-labeled antibody.

Further, according to the present invention, the antigen contained in the sample is allowed to react with a definite amount of the enzyme-labeled antibody in the presence of the immobilized antigen. In other words, according to the present invention, the angigen contained in the sample, on one hand, and the antigen immobilized on the solid phase, on the other hand, are allowed to react with a definite amount of the enzyme-labeled antibody.

After the immunological reaction, a definite amount of the liquid phase is taken out, and its enzyme activity is measured using a dry-type chemical analytical film. In the above immunoassay, a definite amount of an enzyme-labeled antigen or an enzyme-labeled antibody may be incorporated into the porous membrane immobilized with the antibody or antigen before it is allowed to react with the antigen or antibody contained in a sample.

## BRIEF DESCRIPTION OF THE DRAWINGS

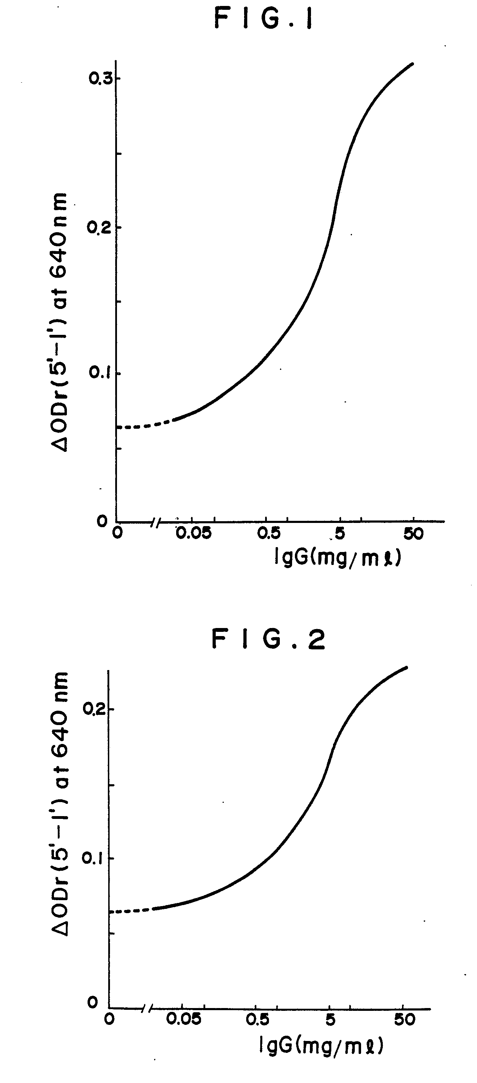

Figures 1 and 2 are graphs showing calibration curves for human IgG obtained in Example 1 and Example 3, respectively.

## DETAILED DESCRIPTION OF THE INVENTION

The porous membrane may be selected from those known in immunological analysis field, including cellulose nitrate porous membrane, cellulose acetate porous membrane, polyamide porous membrane, polytetrafluoroethylene porous membrane, agarose membrane, cellulose filter and glass fiber filter. Preferable porous membranes include cellulose nitrate porous membrane and cellulose acetate porous membrane, prepared by phase-inversion process (for example, described in Robert E. Kesting, "Synthetic Polymeric Membranes", McGraw-Hill, New York, 1971) respectively. Besides, the porous membranes described in Japanese Patent KOKAI No. 18167/1983, particularly from the latter part of page 347 to the preceding part of page 348, are also usable. Glass beads and plastic beads may also be used. But the antibody immobilized to the beads are generally unstable in a dry state, and must be preserved in a stabilizing solution containing serum albumin, etc. On the contrary, the antibody immobilized to the porous membrane is relatively stable even in a dry state, and it can be preserved as it is. The porous membrane has also an advantage that uniform products can be readily mass-produced. Any shape of the porous membrane may be used, including small circular or square piece(s). The volume of the porous membrane is preferably less than the volume of reaction solution so that a definite amount of the liquid phase can readily be taken out.

Prior to conducting EIA, a definite amount of the antibody or antigen is physically and/or chemically immobilized to the porous membrane. In EIA, at least, one of the subject to be measured (analyte), the enzyme-labled substance and the substance immobilized to the porous membrane is antigen, and at least, another one of them is antibody. Thus, the substance to be immobilized may be antibody or antigen, irrespective of whether the analyte is antigen or antibody. One of the antibody and the antigen may be bound to the other substance, and for example, the antigen may be in the state bound to human serum albumin, such as human serum albumin-bound theophylline. The definite amount of the antibody or the like to be immobilized may be the same as a conventional EIA, and decided according to the expected amount of the analyte, the amount of the enzyme-labeled substance and the like. The immobilization method may be a chemical method well known in immunoassay field, while, a physical adsorption method is sufficient. Various immobilization methods are disclosed in the aforementioned "Koso Men-eki Sokutei-Ho". After the immobilization, the nonspecific bound portion of the porous membrane surface is blocked by adding a prescribed amount of an aqueous solution of an immunologically inactive substance such as gelatin or serum albumin (blocker). Then, the porous membrane is washed with a buffer solution containing a surfactant such as Tween 20, a saline solution and the like to obtain the immobilized antibody or the immobilized antigen.

The sample to be assayed may be serum, plasma, urine or the like.

According to the present invention, the antigen contained in the sample is allowed to react with the immobilized antibody in the presence of a definite amount of the enzyme-labeled antigen or antibody, or is allowed to react at first with the immobilized antibody, thereafter with a definite amount of the enzyme-labeled antibody.

Alternatively, according to the present invention, the antibody contained in the sample is allowed to react with the immobilized antigen in the presence of a definite amount of the enzyme-labeled antibody.

Further, according to the present invention, the antigen contained in the sample is allowed to react with a definite amount of the enzyme-labeled antibody in the presence of the immobilized antigen. In other words, according to the present invention, the angigen contained in the sample, on one hand, and the antigen immobilized on the solid phase, on the other hand, are allowed to react with a definite amount of the enzyme-labeled antibody. The reaction may be carried out according to a known EIA method.

If an antigen in a sample is to be determined by using a competitive method, such as the solid phase method, antigen-antibody reaction is allowed to occur among the antigen contained in the sample, a definite amount of the enzyme-labeled antigen or antibody and a definite amount of the immobilized antibody or antigen, competitively. The reaction is conducted at 0 to about 37°C for several tens minutes to one day, preferably at 10 to 25° C for 1 to 3 hours. The antigen contained in the sample and the labeled antigen or the immobilized antigen competitively reacts with the immobilized antibody or the labeled antibody, respectively, and as a result, the amount of the enzyme-labeled antigen or antibody bound to solid phase is correlated to the amount of the antigen in the sample.

If an antibody in a sample is to be determined by using a competitive method, the antibody contained in the sample and a definite amount of the enzyme-labeled antibody are allowed to react with a definite amount of the immobilized antigen, competitively. The reaction temperature and the reaction time may be the same as the above case of measuring antibody. The antibody contained in the sample and the labeled antibody competitively reacts with the immobilized antigen, and as a result, the amount of the enzyme-labeled antibody bound to solid phase correlates to the amount of the antibody in the sample.

If an antigen in a sample is to be determined by using a non-competitive method, such as sandwich method, the antigen contained in the sample is allowed to react with a sufficient amount of the immobilized antibody at 0 to about 37° C for several tens minutes to one day, preferably at 10 to 25° C for 2 to 10 hours. Then, the enzyme labeled antibody is added, and allowed to react at 0 to 37° C for several tens minutes to one day, preferably at 10 to 25° C for 2 to 10 hours.

The enzyme used as label may be selected from the enzymes used in known EIA, and representative enzymes are glucose oxidase (GOD), peroxidase (POD), alkaline phosphatase (ALP), $\beta$-galactosidase ($\beta$-GAL) and the like. The enzyme-labeled antigens and antibodies may be those used in conventional EIA, and they can be prepared, for example, according to a method disclosed in the aforementioned "Koso Men-eki Sokutei-Ho".

A definite amount of the enzyme-labeled antigen or antibody may be incorporated into the porous membrane where a definite amount of the antibody or antigen has been immobilized, respectively, prior to conducting the antigen-antibody reaction with the antigen or antibody contained in a sample. In this procedure the antigen or antibody in a sample can be measured merely by adding the sample to the porous membrane, even in the sandwich method. The labeled antigen or antibody is preferably incorporated after the blocking of the non-specific bound portion followed by washing. The incorporation may be carried out by impregnating a solution of the labeled substance, followed by drying. The total reaction time is 1 hour to 1 day at 0 to about 37° C, preferably 1 to 3 hours at 10 to 25° C, irrespective of competitive method, or non-competitive method such as sandwich method.

After the above reaction, a definite amount of the liquid phase is taken out, and the enzyme activity of the liquid phase is measured by using a dry-type chemical analytical film.

The dry-type chemical analytical film may be prepared according to the disclosures of U.S.P. 3,992,158, Japanese Patent KOKAI 168159/1982 and 175199/1987. The dry-type chemical analytical film may be composed of single layer or multiple layers. In the latter case, respective layers are preferably integrated. However, each layer or layer group may be separable, and liquid can temporarily flow into the space therebetween. The chemical analytical film contains a substrate of the enzyme reaction and a reagent for detecting a product of the enzyme reaction in a water-permeable layer. Instead of the above reagent, another reagent capable of determining a reaction component of the enzyme reaction may be incorporated. The enzyme reaction of a reagent is conveniently detected photometrically by utilizing the coloration or decoloration due to the reagent, however, it may also be detected electrically or magnetically. The substrate may be a self-developing type substrate such as a para-nitrophenyl phosphate, and in such a case, the above reagent may be omitted. The substrate may be incorporated into the same layer as or a different layer from the reagent-containing layer. When the chemical analytical film is an integral multilayer analytical film, it has preferably a spreading layer for spreading the sample. The spreading layer preferably spreads the sample solution supplied thereto to the area in proportion to the supplied volume of the sample solution. One or both of the substrate and the reagent may be incorporated into the spreading layer. The integral multilayer analytical film has preferably a light-transmissive support. The support may be water-permeable, but it is preferably water-impermeable. The light-transmissive water-impermeable support is preferably disposed on the opposite side to the spreading layer.

In the method of the invention, the enzyme label amount of the free portion remaining in the liquid phase is measured by rate assay using the dry-type chemical analytical film containing all reagent necessary for the measurement of the enzyme activity. That is, after the finishment of the competitive reaction, a definite amount of the liquid phase is spotted onto the dry-type chemical analytical film, and the variation of the reflection optical density is measured using a reflection densitometer in a prescribed time range. The reaction rate is determined from the variation rate of the optical density, and the enzyme activity is calculated.

From the enzyme activity, the antigen concentration of the sample is determined.

Representative embodiments of the immunoassay of the invention are described below.

(a) An antigen contained in a sample and a definite amount of the enzyme-labeled antigen not less than the undermentioned immobilized antibody are allowed to react with an immobilized antibody where a definite amount of the antibody is physically and/or chemically immumobilized to a porous membrane, a definite amount of the liquid phase is taken out, and the enzyme activity of the liquid phase is measured by using a dry-type chemical analytical film.

(b) An antigen contained in a sample and an immobilized antigen obtained by immobilizing a definite amount of the antigen physically and/or chemically to a porous membrane are allowed to react with a definite amount of the enzyme-labeled antibody not less than the above immobilized antigen, a definite amount of the liquid phase is taken out, and the enzyme activity of the liquid phase is measured by using a dry-type chemical analytical film.

(c) An antibody contained in a sample and a definite amount of the enzyme-labeled antibody not less than the undermentioned immobilized antigen are allowed to react with the immobilized antigen, a definite amount of the liquid phase is taken out, and the enzyme activity of the liquid phase is measured by using a dry-type chemical analytical film.

(d) An antigen contained in a sample is allowed to react with the immobilized antibody much more than the antigen in a sample, the antigen bound to the immobilized antibody is further allowed to react with a definite amount of the enzyme-labeled antibody not less than the above immobilized antibody, a definite amount of the liquid phase is taken out, and the enzyme activity of the liquid phase is measured by using a dry-type chemical analytical film.

(e) The embodiment (a) wherein the enzyme-labeled antigen is incorporated in the porous membrane where the antibody has been immobilized, prior to be allowed to react with the antigen contained in the sample.

(f) The embodiment (b) wherein the enzyme-labeled antibody is incorporated in the porous membrane where the antigen has antigen contained in the sample.

(g) The embodiment (c) wherein the enzyme-labeled antibody is incorporated in the porous membrane where the antigen has been immobilized, prior to be allowed to react with the antibody contained in the sample.

(h) The embodiment (d) wherein the enzyme-labeled antibody is incorporated in the porous membrane where the antibody has been immobilized, prior to be allowed to react with the antigen contained in the sample.

## EXAMPLES

### Example 1

(1) Preparation of Analytical Film for Measuring GOD Activity.

The support employed was a transparent polyethylene terephthalate (PET) having a thickness of 180 μm of which the surface was made hydrophilic. The following coating solution was appied thereon at a rate of 155 ml/m², and dried to form a dye-forming layer.

Gelatin       160 g
Water        1,000 g
Peroxidase        150,000 units
Leuco dye*       2.4 g
Bis(vinylsulfonylmethylcarbonyl) amino methane
    1.6 g
*       2-(4-hydroxy-3,5-dimethoxyphenyl)-4-       4-(dimethylamino)phenyl -5-phenetylimidazole

Subsequently, the following coating solution was applied further thereon at a rate of 62.5 ml/m², and dried to from a binding layer.
Gelatin       60 g
Water       1,140 g
Glucose       40 g

The above binding layer was moistened with about 30 g/m² of water, and a tricot fabric made by knitting 50 deniers PET spun yarn using 36 gauge was pressed on it to laminate it as the spreading layer, followed by drying. Then, the following coating solution was applied thereon at a rate of 120 ml/m², and dried to complete an analytical film for measuring GOD activity.
5 w/w % Aqueous polyvinyl pyrrolidone solution
    200 g
Glucose       20 g
N-2-hydroxyethylpiperazine-2-ethanesulfonic acid
    9.4 g
Adjusted to pH 6.5 by adding 2N NaOH

(2) Determination of Human IgG

(a) Immobilization of Antibody

Anti-human IgG mouse antibody was obtained by immunizing a mouse according to a conventional manner, and dissolved in 0.02 M phosphate buffer solution (PBS) of pH 7.4 in a concentration of 1,000 μg/ml. 2.5 μl of the antibody solution was impregnated into a circular disc of cellulose acetate porous membrane ("Microfilter FM 300", Fuji Photo Film Co., Ltd.) having a diameter of 4.5 mm, and dried for 10 minutes. In order to block the non-specific bound portion of the porous membrane 2.5 μl of PBS containing 1 % of human serum albumin was further impregnated thereinto, and dried for 10 minutes. The porous membrane was washed with PBS containing 0.05 % of a surfactant ("Tween 20", Tokyo Kasei Kogyo Co., Ltd.) to complete an antibody-immobilized porous membrane.

(b) Preparation of IgG Calibration Curve

Each 25 μl of a PBS containing 0, 0.05, 0.5, 5 or 50 mg/ml of human IgG and 0.1 % of human serum albumin was placed in a test tube, and 25 μl of a glucose oxidase-labeled human IgG in PBS having an activity of 200 GOD units/l and 0.1 % of human serum albumin was added to each test tube. The GOD-labeled IgG was prepared by the maleimide method described in the aforementioned "Koso Men-eki Sokutei-Ho". Each 1 piece of the above antibody-immobilized porous membrane was put into the test tube, and incuvated for 1 hour at room temperature. Each 10 μl of the reaction solution was taken out by using a micropipette, and spotted onto the analytical film for measuring GOD activity in the reaction solution. The variation of the reflection optical density (ΔODr) was measured from 1 minute to 5 minutes after the spotting by reflection photometry, and the calibration curve shown in Figure 1 was obtained. A human IgG concentration of a sample can be determined in the range from 0.5 to 50 mg/ml by using the calibration curve.

(c) Determination of Human IgG

2.5 μl of GOD-labeled IgG solution having an activity of 2,000 GOD units/l was immersed into the antibody-immobilized porous membrane, and rapidly dried. One piece of this antibody-immobilized porous membrane was placed in a test tube. Each 50 μl of various sample solutions of PBS containing unknown concentration of human IgG and 0.1 % of human serum albumin was added to the test tube, and incubated at room temperature for 1 hour. 10 μl of the reaction solution was taken out from each test tube, and the GOD activity of the reaction solution was measured using the analytical film prepared in (1) in the same manner as described in (2). A human IgG concentration was determined for various samples in the range from 0.5 to 50 mg/ml.

Example 2

Using 100 μg/ml of anti-human ferritin antibody instead of anti-IgG mouse antibody, another antibody-immobilized porous membrane was prepared.

Each sample solution containing human ferritin and 0.1 % of human serum albumin was added to the porous membrane, and incubated at room temperature for 2 hours. The porous membrane was washed with 0.05 % of a surfactant solution ("Tween 20"). A GOD-labeled anti-human ferritin antibody solution having 200 U/l of GOD activity was added, and further incubated for 2 hours. The GOD-labeled anti-human ferritin antibody was prepared by the maleimide method described in the aforementioned "Koso Men-eki Sokutei-Ho". The GOD activity of the reaction solution was measured in a similar manner to Example 1, and human ferritin was determined for various samples in the range from 10 to 600 ng/ml.

Example 3

An anti-human IgG mouse antibody was immobilized to a circular cellulose acetate porous membrane ("Microfilter FM300", Fuji Photo Film Co., Ltd.) having a diameter of 4.5 mm in the same manner as Example 1. 2.5 μl of GOD-labeled IgG solution having an activity of 2,000 GOD units/l was impregnated into the antibody-immobilized porous membrane washed with PBS containing 0.05 % of a surfactant ("Tween 20"), and rapidly dried to obtain an antibody-immobilized porous membrane containing enzyme-labeled antigen.

Each 50 μl of a PBS containing 0, 0.05, 0.5, 5 or 50 mg/ml of human IgG and 0.1 % of human serum albumin was placed in a test tube. Each 1 piece of the above antibody-immobilized porous membrane containing enzyme-labeled antigen was put into the test tube, and a calibration curve shown in Figure 2 was obtained by conducting the same manner as Example 1 (b). A human IgG concentration of a sample can be determined in the range from 0.5 to 50 mg/ml by using the calibration curve.

Subsequently, one piece of the antibody-immobilized porous membrane containing enzyme-labeled antigen was placed in each test tube, and concentration of human IgG in various sample solutions of PBS containing and 0.1 % of human serum albumin was measured in the same manner as Example 1 (c). As a result, a human IgG concentration was determined for various samples in the range from 0.5 to 50 mg/ml.

Example 4

Anti-human ferritin antibody was obtained by immunizing a mouse according to a conventional manner, and it was immobilized to a circular disc of cellulose acetate porouse membrane ("Microfilter FM300") in the same manner as Example 1 (2) (a). 2.5 μl of GOD-labeled anti-human ferritin antibody PBS having an activity of 2,000 GOD units/l containing 0.1 % human serum albumin was impregnated in the above antibody-immobilized porous membrane washed with PBS containing 0.05 % of a surfactant ("Tween 20"), and rapidly dried. The GOD-labeled anti-human ferritin antibody was prepared according to the maleimide method described in the aforementioned "Koso Men-eki Sokutei-Ho".

One piece of the above antibody-immobilized porous membrane containing enzyme-labeled antibody was put into a sample solution which was a PBS containing human ferritin and 0.1 % human serum albumin, and incubated at room temperature for 2 hours. The 10 μl of the reaction solution was taken out, and the GOD activity was measured in the same manner as Example 1 (3). As a result, a human ferritin concentration was determined for various samples in the range from 10 to 600 ng/ml.

**Claims**

1. In a heterogeneous immunoassay using an enzyme label for determining an antigen or antibody in a sample liquid, the improvement comprising allowing the antigen or antibody contained in the sample to react with an immobilized antibody or antigen, respectively where a definite amount of the antibody or antigen is physically and/or chemically immobilized to a porous membrane in the presence of a definite amount of an enzyme-labeled antigen or antibody, respectively, taking out a definite amount of the liquid phase containing free antigen or antibody after the reaction, and measuring the enzyme activity of said liquid phase by using a dry-type chemical analytical film.

2. The immunoassay for determining an antigen in a sample of claim 1 wherein the antigen contained in the sample and the enzyme-labeled antigen are allowed to react with said immobilized antibody, said amount of enzyme-labeled antigen being not less than the amount of said immobilized antibody.

3. The immunoassay for determining an antigen in a sample of claim 1 wherein the antigen contained in the sample and said immobilized antigen are allowed to react with the enzyme-labeled antibody, said amount of enzyme-labeled antibody being not less than the amount of said immobilized antigen not less than said immobilized antigen.

4. The immunoassay for determining an antibody in a sample of claim 1 wherein the antibody contained in the sample and the enzyme-labeled antibody are allowed to react with said immobilized antigen, said amount of enzyme-labeled antibody being not less than the amount of said immobilized antigen.

5. The immunoassay for determining an antigen in a sample of claim 1 wherein the antigen contained in the sample is allowed to react with said immobilized antibody the amount of which is much greater than that of the antigen in a sample, the antigen not bound to the the antibody is removed by washing, and the antigen bound to the immobilized antibody through the reaction is further allowed to react with the enzyme-labeled antibody present in an amount not less than that of said immobilized antibody.

6. The immunoassay for determining an antigen or an antibody in a sample of claim 1 wherein said enzyme-labeled antigen or antibody is incorporated in said porous membrane where the antibody or antigen, respectively has been immobilized, before the antigen or antibody in the sample is allowed to react with the antigen or antibody contained in the sample.

7. The immunoassay for determining an antigen in a sample of claim 6 wherein the antigen contained in the sample is allowed to react with said immobilized antibody in competition with the enzyme-labeled antigen.

8. The immunoassay for determining an antigen in a sample of claim 6 wherein the antigen contained in the sample is allowed to react with the enzyme-labeled antibody in competition with the immobilized antigen.

9. The immunoassay for determining an antibody in a sample of claim 6 wherein the antibody contained in the sample is allowed to react with the immobilized antigen in competition with the enzyme-labeled antibody.

10. The immunoassay for determining an antigen in a sample of claim 6 wherein the antigen contained in the sample is allowed to react with said immobilized antibody and the enzyme-labeled antibody incorporated in the porous membrane.

EP 0 308 704 A1

F I G . 1

F I G . 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | GB - A - 2 199 946 (PALL COR-PORATION) <br> * Abstract; examples 1-7 * <br> -- | 1 | G 01 N 33/545 <br> G 01 N 33/541 <br> G 01 N 33/535 <br> G 01 N 33/52 |
| P,X | US - A - 4 693 985 (DEGEN et al.) <br> * Abstract * <br> -- | 1 | |
| A | GB - A - 4 604 347 (ARAI et al.) <br> * Abstract; examples * <br> ---- | 1 | |

**TECHNICAL FIELDS SEARCHED (Int. Cl.4)**

G 01 N 33/00

The present search report has been drawn up for all claims

| Place of search | Date of completion of the search | Examiner |
|---|---|---|
| VIENNA | 07-12-1988 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82